(19) Europäisches Patentamt
European Patent Office
Office européen des brevets

(11) EP 4 180 441 A1

(12) EUROPEAN PATENT APPLICATION
published in accordance with Art. 153(4) EPC

(43) Date of publication:
17.05.2023 Bulletin 2023/20

(21) Application number: 21837251.4

(22) Date of filing: 07.07.2021

(51) International Patent Classification (IPC):
C07H 15/08 (2006.01) C07H 17/04 (2006.01)
C07H 1/00 (2006.01) C08G 18/32 (2006.01)
C08G 65/26 (2006.01) C08G 18/48 (2006.01)
C08G 18/10 (2006.01) C08G 18/64 (2006.01)
C09J 175/04 (2006.01)

(52) Cooperative Patent Classification (CPC):
C07H 1/00; C07H 15/08; C07H 17/04; C08G 18/10; C08G 18/32; C08G 18/48; C08G 18/64; C08G 65/26; C09J 175/04

(86) International application number:
PCT/KR2021/008638

(87) International publication number:
WO 2022/010252 (13.01.2022 Gazette 2022/02)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 08.07.2020 KR 20200083991

(71) Applicant: Samyang Corporation
Jongno-gu
Seoul 03129 (KR)

(72) Inventors:
- SONG, Gwang Seok
Jeonju-si Jeollabuk-do 55021 (KR)
- RYU, Hoon
Daejeon 34127 (KR)
- NOH, Jae Guk
Daejeon 34185 (KR)
- YOO, Seung Hyun
Daejeon 34637 (KR)
- JANG, Min Jung
Bucheon-si Gyeonggi-do 14472 (KR)

(74) Representative: Schrell, Andreas et al
Gleiss Große Schrell und Partner mbB
Patentanwälte Rechtsanwälte
Leitzstrasse 45
70469 Stuttgart (DE)

# (54) ALKYLENE-OXIDE-ADDED POLYOL COMPOSITION, POLYURETHANE USING SAME, AND HOT-MELT ADHESIVE COMPRISING SAME

(57) The present invention relates to an alkylene-oxide-added polyol composition, a polyurethane using same, and a hot-melt adhesive comprising same, and, more specifically, to an alkylene oxide-added polyol composition, a polyurethane using same, and a hot-melt adhesive comprising same, the composition being prepared by performing, in a specific content ratio, an addition reaction between an alkylene oxide and an anhydrosugar alcohol composition comprising a) a monoanhydrosugar alcohol, b) a dianhydrosugar alcohol, c) a polysaccharide alcohol, d) an anhydrosugar alcohol derived from a polysaccharide alcohol, and e) a polymer of at least one of a) to d), and thus can increase the amount of bio components, improves adhesive strength, and enables a polyurethane to be prepared at a cost lower than that of when using petroleum polyols and other biopolyols.

EP 4 180 441 A1

## Description

[TECHNICAL FIELD]

The present invention relates to an alkylene oxide-added polyol composition, a polyurethane using the same, and a hot-melt adhesive comprising the same, and more specifically, to an alkylene oxide-added polyol composition capable of improving bio-content, adhesiveness and producing polyurethane at a lower price than petroleum-based polyol and other bio-polyols, prepared by an addition reaction of an anhydrosugar alcohol composition comprising a) monoanhydrosugar alcohol; b) dianhydrosugar alcohol; c) polysaccharide alcohol; d) anhydrosugar alcohol derived from a polysaccharide alcohol; and e) a polymer of one or more of a) to d) and an alkylene oxide with specific contents, a polyurethane using the same, and a hot-melt adhesive comprising the same.

[BACKGROUND ART]

Recently, as awareness of environmental issues has grown, interest in polyurethane hot-melt materials and the need for improvement of properties are increasing. In general, since hot-melt adhesives are applied after being melted by heat, the emission of volatile organic solvents is very low, so their use as eco-friendly adhesives is increasing, and attempts have been made to improve adhesiveness or other physical properties by using various components or additives (for example, Korean Patent Laid-open Publication No. 10-2013-0119850 and Korean Patent No. 10-1370442 or 10-1709909, etc.).

Hydrogenated sugar (also known as "sugar alcohol") refers to a compound obtained by adding hydrogen to the reducing terminal group of a saccharide. Generally, it has the formula $HOCH_2(CHOH)_nCH_2OH$ (wherein n is an integer of 2 to 5) and is classified into tetritol, pentitol, hexitol and heptitol (having 4, 5, 6 and 7 carbon atoms, respectively) depending on the number of carbon atoms. Among them, hexitol having 6 carbon atoms includes sorbitol, mannitol, iditol, galactitol and the like, and sorbitol and mannitol are particularly useful substances.

Anhydrosugar alcohol is a substance formed by removing one or more water molecules from the inside of hydrogenated sugar. When one water molecule is removed, it has the form of tetraol with four hydroxyl groups in the molecule, and when two water molecules are removed, it has a diol form with two hydroxyl groups in the molecule, and it can be prepared using hexitol derived from starch (for instance, Korean Patent No. 10-1079518 and Korean Patent Laid-open Publication No. 10-2012-0066904). Since anhydrosugar alcohol is an eco-friendly substance derived from renewable natural resources, there has been much interest in it for a long time, and studies of the production method have been carried out. Among these anhydrosugar alcohols, isosorbide prepared from sorbitol presently has the largest industrial application range.

Anhydrosugar alcohol is widely used in the treatment of cardiac and vascular diseases, adhesives for patches, drugs for mouthwash and the like, solvents for compositions in the cosmetics industry and emulsifiers in the food industry. In addition, it is possible to increase the glass transition temperature of a polymer such as polyester, PET, polycarbonate, polyurethane and epoxy resin, and to improve the strength of these materials, and it is also very useful in the plastics industry such as bioplastics since it is an eco-friendly material derived from natural materials. It is also known to be used as adhesives, eco-friendly plasticizers, biodegradable polymers and an eco-friendly solvent for water-soluble lacquers.

As such, anhydrosugar alcohol has attracted a great deal of attention due to its versatility, and its use in industry is increasing.

Conventional commercialized hot-melt adhesives lack adhesive strength, and as adhesives derived from petroleum resources, improvement is required in terms of environmental friendliness.

[CONTENTS OF THE INVENTION]

[PROBLEMS TO BE SOLVED]

The purpose of the present invention is to provide an alkylene oxide-added polyol composition capable of improving bio content, adhesion and producing polyurethane at a lower price than petroleum-based polyol and other bio-polyols, prepared by an addition reaction of an anhydrosugar alcohol composition comprising a) monoanhydrosugar alcohol; b) dianhydrosugar alcohol; c) polysaccharide alcohol; d) anhydrosugar alcohol derived from a polysaccharide alcohol; and e) a polymer of one or more of a) to d) and an alkylene oxide with specific contents, a polyurethane using the same, and a hot-melt adhesive comprising the same.

[TECHNICAL MEANS]

In order to achieve the technical purpose, in the first aspect, the present invention provides a polyol composition

prepared by addition reaction of 100 parts by weight of an anhydrosugar alcohol composition and more than 50 parts by weight to less than 4,000 parts by weight of an alkylene oxide, wherein the anhydrosugar alcohol composition comprises a) monoanhydrosugar alcohol; b) dianhydrosugar alcohol; c) polysaccharide alcohol represented by the following Formula 1; d) anhydrosugar alcohol derived from the polysaccharide alcohol represented by the following Formula 1; and e) a polymer of one or more of a) to d):

[Formula 1]

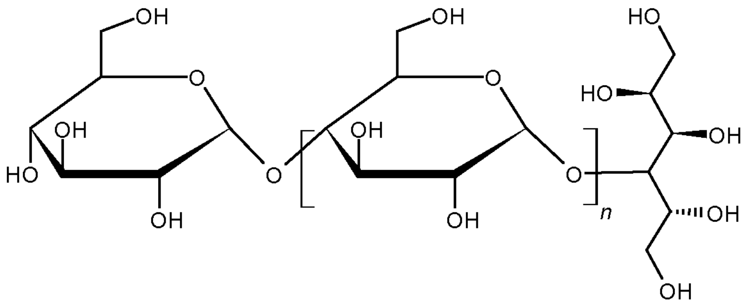

In Formula 1, n is an integer of 0 to 4.

In another aspect, the present invention provides a polyurethane prepolymer prepared by a reaction of the polyol composition of the present invention with a polyisocyanate.

In another aspect, the present invention provides a chain-extended polyurethane prepared by a reaction of the polyurethane prepolymer of the present invention with a chain extender.

In another aspect, the present invention provides a method for preparing a chain-extended polyurethane comprising (1) preparing a polyurethane prepolymer by reacting the polyol composition of the present invention with a polyisocyanate; and (2) reacting the polyurethane prepolymer with a chain extender.

In another aspect, the present invention provides a hot-melt adhesive comprising the chain-extended polyurethane of the present invention.

[EFFECT OF THE INVENTION]

According to the present invention, since the polyol composition prepared by adding alkylene oxide to the internal dehydration of hydrogenated sugars and/or by-products generated after the production of various sugars can be used as a polyol for producing polyurethane, it is possible to improve bio-content, adhesiveness and produce polyurethane at a lower price than petroleum-based polyol and other bio-polyols while resolving the cost and environmental pollution problems that arise when the by-products are treated as industrial waste (incineration, landfill, etc.).

[CONCRETE MODE FOR CARRYING OUT THE INVENTION]

The present invention is explained in more detail below.

The polyol composition of the present invention is prepared by addition reaction of 100 parts by weight of an anhydrosugar alcohol composition and more than 50 parts by weight to less than 4,000 parts by weight of an alkylene oxide, wherein the anhydrosugar alcohol composition comprises a) monoanhydrosugar alcohol; b) dianhydrosugar alcohol; c) polysaccharide alcohol represented by the following Formula 1; d) anhydrosugar alcohol derived from the polysaccharide alcohol represented by the following Formula 1; and e) a polymer of one or more of a) to d):

[Formula 1]

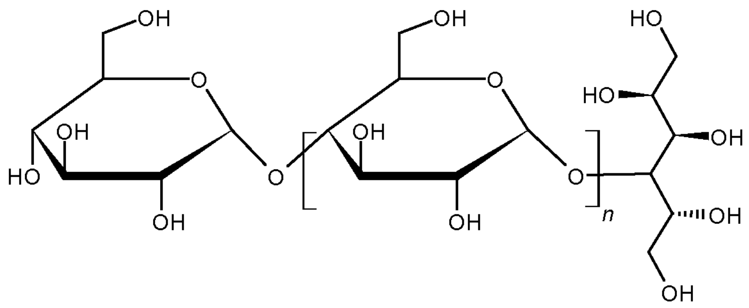

In Formula 1, n is an integer of 0 to 4.

Anhydrosugar alcohol can be produced by dehydrating natural product-derived hydrogenated sugar. Hydrogenated sugar (also known as "sugar alcohol") refers to a compound obtained by adding hydrogen to the reducing terminal group of a saccharide. Generally, it has the formula $HOCH_2(CHOH)_nCH_2OH$ (wherein n is an integer of 2 to 5) and is classified into tetritol, pentitol, hexitol and heptitol (having 4, 5, 6 and 7 carbon atoms, respectively) depending on the number of carbon atoms. Among them, hexitol having 6 carbon atoms includes sorbitol, mannitol, iditol, galactitol and the like, and sorbitol and mannitol are particularly useful substances.

One or more, preferably two or more, more preferably all of a) monoanhydrosugar alcohol; b) dianhydrosugar alcohol; c) polysaccharide alcohol represented by the following Formula 1; d) anhydrosugar alcohol derived from the polysaccharide alcohol represented by the following Formula 1; and e) a polymer of one or more of a) to d) comprised in the anhydrosugar alcohol composition of the present invention can be obtained by hydrogenating a glucose-containing saccharide composition (e.g., a saccharide composition comprising disaccharides or higher polysaccharides including glucose, mannose, fructose and maltose) to prepare a hydrogenated sugar composition, heating the obtained hydrogenated sugar composition under an acid catalyst to a dehydration reaction by heating and conducting thin-film-distillation of the obtained dehydration reaction product. More specifically, all of a) to e) comprised in the anhydrosugar alcohol composition of the present invention may be by-products remaining after obtaining a thin-film distillate by thin-film-distillation of the obtained dehydration reaction product.

Monoanhydrosugar alcohol is anhydrosugar alcohol formed by removing one water molecule from the inside of hydrogenated sugar and has a tetraol form with four hydroxyl groups in the molecule.

In the present invention, the type of a) monoanhydrosugar alcohol is not particularly limited, but may be preferably monoanhydrosugar hexitol, and more specifically, 1,4-anhydrohexitol, 3,6-anhydrohexitol, 2,5-anhydrohexitol, 1,5-anhydrohexitol, 2,6-anhydrohexitol or a mixture of two or more thereof.

Dianhydrosugar alcohol is anhydrosugar alcohol formed by removing two water molecules from the inside of hydrogenated sugar, has a diol form with two hydroxyl groups in the molecule and can be prepared by using hexitol derived from starch. Since dianhydrosugar alcohol is an eco-friendly material derived from renewable natural resources, research on its manufacturing method has been conducted with much interest for a long time. Among these dianhydrosugar alcohols, isosorbide prepared from sorbitol currently has the widest range of industrial applications.

In the present invention, the type of b) dianhydrosugar alcohol is not particularly limited, but may be preferably dianhydrosugar hexitol, more specifically, it may be 1,4:3,6-dianhydrohexitol. The 1,4:3,6-dianhydrohexitol may be isosorbide, isomannide, isoidide or a mixture of two or more thereof.

In the present invention, c) the polysaccharide alcohol represented by the following Formula 1 can be prepared by hydrogenation of disaccharides or higher polysaccharides including maltose.

[Formula 1]

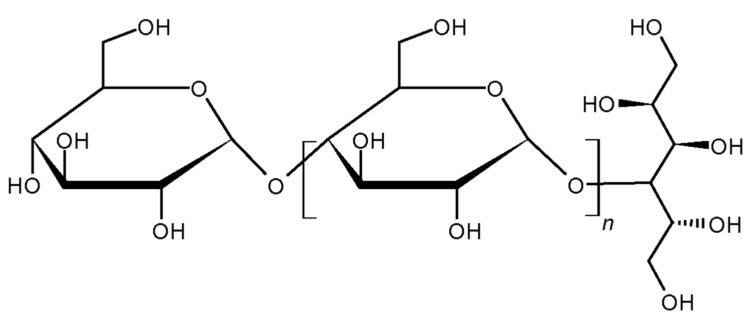

In Formula 1, n is an integer of 0 to 4.

In the present invention, d) anhydrosugar alcohol derived from polysaccharide alcohol represented by Formula 1 may be selected from a compound represented by the following Formula 2, a compound represented by the following Formula 3 or a mixture thereof:

[Formula 2]

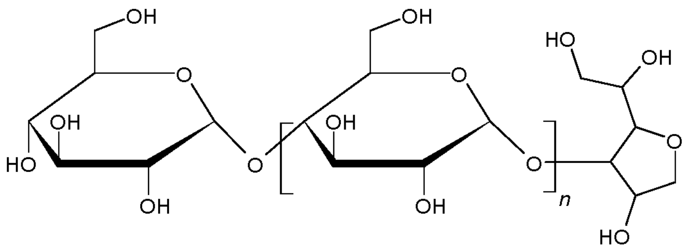

[Formula 3]

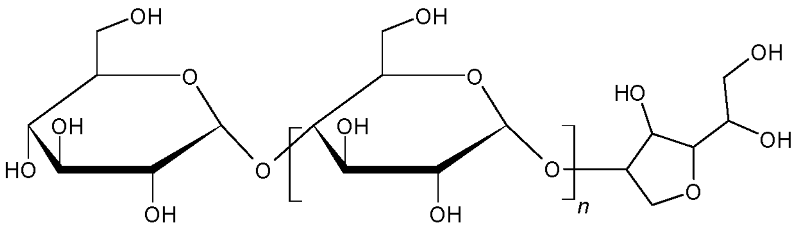

In Formulae 2 and 3, each of n is independently an integer of 0 to 4.

In the present invention, e) the polymer of one or more of a) to d) may comprise at least one selected from the group consisting of condensation polymers prepared from the following condensation reaction. In the following condensation reaction, the condensation position and condensation sequence between the monomers are not particularly limited, and may be selected without limitation within a range that could be commonly predicted by a person skilled in the art:

- condensation reaction of monoanhydrosugar alcohol,
- condensation reaction of dianhydrosugar alcohol,
- condensation reaction of the polysaccharide alcohol represented by Formula 1,
- condensation reaction of anhydrosugar alcohol derived from polysaccharide alcohol represented by Formula 1,
- condensation reaction of monoanhydrosugar alcohol and dianhydrosugar alcohol,
- condensation reaction of monoanhydrosugar alcohol and polysaccharide alcohol represented by Formula 1,
- condensation reaction of monoanhydrosugar alcohol and anhydrosugar alcohol derived from polysaccharide alcohol represented by Formula 1,
- condensation reaction of dianhydrosugar alcohol and polysaccharide alcohol represented by Formula 1,
- condensation reaction of dianhydrosugar alcohol and anhydrosugar alcohol derived from polysaccharide alcohol represented by Formula 1,
- condensation reaction of polysaccharide alcohol represented by Formula 1 and anhydrosugar alcohol derived from polysaccharide alcohol represented by Formula 1,
- condensation reaction of monoanhydrosugar alcohol, dianhydrosugar alcohol and polysaccharide alcohol represented by Formula 1,
- condensation reaction of monoanhydrosugar alcohol, dianhydrosugar alcohol and anhydrosugar alcohol derived from polysaccharide alcohol represented by Formula 1,
- condensation reaction of monoanhydrosugar alcohol, polysaccharide alcohol represented by Formula 1 and anhydrosugar alcohol derived from polysaccharide alcohol represented by Formula 1,
- condensation reaction of dianhydrosugar alcohol, polysaccharide alcohol represented by Formula 1 and anhydrosugar alcohol derived from polysaccharide alcohol represented by Formula 1, or
- condensation reaction of monoanhydrosugar alcohol, dianhydrosugar alcohol, polysaccharide alcohol represented

by Formula 1 and anhydrosugar alcohol derived from polysaccharide alcohol represented by Formula 1.

In one embodiment, in the anhydrosugar alcohol composition of the present invention, 0.1 to 20 wt%, specifically 0.6 to 20 wt%, more specifically 0.7 to 15 wt% of the a) monoanhydrosugar alcohol may be comprised, 0.1 to 28 wt%, specifically 1 to 25 wt%, more specifically 3 to 20 wt% of the b) dianhydrosugar alcohol may be comprised, the total content of c) polysaccharide alcohol represented by Formula 1 and d) anhydrosugar alcohol derived from polysaccharide alcohol represented by Formula 1 may be 0.1 to 6.5 wt%, specifically 0.5 to 6.4 wt%, more specifically 1 to 6.3 wt%, and 55 to 90 wt%, specifically 60 to 89.9 wt%, more specifically 70 to 89.9 wt% of e) one or more polymers of a) to d) based on the total weight of the composition, but they are not particularly limited thereto.

In the present invention, the content of the alkylene oxide which is added to the anhydrosugar alcohol composition may be more than 50 parts by weight, 55 parts by weight or more, 60 parts by weight or more, 70 parts by weight or more, 80 parts by weight or more, 90 parts by weight or more, or 100 parts by weight or more, and less than 4,000 parts by weight, 3,900 parts by weight or less, 3,700 parts by weight or less, 3,500 parts by weight or less, 3,200 parts by weight or less, or 3,000 parts by weight or less per 100 parts by weight of the anhydrosugar alcohol composition.

In one embodiment, the amount of alkylene oxide which is added to the anhydrosugar alcohol composition may be greater than 50 parts by weight and less than 4,000 parts by weight, 60 to 3,900 parts by weight, 80 to 3,500 parts by weight or 90 to 3,200 parts by weight per 100 parts by weight of the anhydrosugar alcohol composition. If the content of the added alkylene oxide is 50 parts by weight or less, the soft portion imparting flexibility in the hot-melt specimen prepared using the polyol composition of the present invention is too small (the alkylene oxide functions to impart soft characteristics) and cohesive peeling, in which the hot-melt adhesive itself is broken, may occur. If the content of the added alkylene oxide is 4,000 parts by weight or more, the urethane groups imparting adhesive strength in the hot-melt specimen are too small (urethane produced by bonding polyol and isocyanate functions to impart adhesive strength), and surface peeling (the adhesive interface peels off) may occur.

In one embodiment, the alkylene oxide may be a C2-C8 linear or C3-C8 branched alkylene oxide, and more specifically, ethylene oxide, propylene oxide or a combination thereof.

In the polyol composition of the present invention, the number average molecular weight (Mn) of the anhydrosugar alcohol composition may be 193 or more, 195 or more, 200 or more, 202 or more, 205 or more, or 208 or more. In addition, the number average molecular weight (Mn) of the anhydrosugar alcohol composition of the present invention may be 1,589 or less, 1,560 or less, 1,550 or less, 1,520 or less, 1,500 or less, 1,490 or less, or 1,480 or less.

In one embodiment, the number average molecular weight (Mn) of the anhydrosugar alcohol composition may be 193 to 1,589, specifically 195 to 1,550, more specifically 200 to 1,520, more specifically 202 to 1,500, and much more specifically 205 to 1,490. If the number average molecular weight of the anhydrosugar alcohol composition is less than 193, polyurethane polymerization may be difficult when producing polyurethane foam using the same, and if the number average molecular weight of the anhydrosugar alcohol composition is more than 1,589, the adhesive strength of the hot-melt adhesive using the polyurethane prepared by applying the polyol composition prepared using the same may be poor.

In the polyol composition of the present invention, the polydispersity index (PDI) of the anhydrosugar alcohol composition may be 1.13 or more, 1.15 or more, 1.20 or more, 1.23 or more, or 1.25 or more. In addition, the polydispersity index (PDI) of the anhydrosugar alcohol composition of the present invention may be 3.41 or less, 3.40 or less, 3.35 or less, 3.30 or less, 3.25 or less, 3.22 or less, or 3.19 or less.

In one embodiment, the polydispersity index (PDI) of the anhydrosugar alcohol composition may be 1.13 to 3.41, specifically 1.13 to 3.40, more specifically 1.15 to 3.35, more specifically 1.20 to 3.35, and much more specifically 1.23 to 3.22. If the polydispersity index of the anhydrosugar alcohol composition is less than 1.13 or more than 3.41, the adhesive strength of the hot-melt adhesive using the polyurethane prepared by applying the polyol composition prepared using the same may be poor.

In the polyol composition of the present invention, the average number of -OH groups per molecule in the anhydrosugar alcohol composition may be 2.54 or more, 2.60 or more, 2.65 or more, 2.70 or more, 2.75 or more, or 2.78 or more. Further, the average number of -OH groups per molecule in the anhydrosugar alcohol composition of the present invention may be 21.36 or less, 21.30 or less, 21.0 or less, 20.5 or less, 20.0 or less, 19.95 or less, or 19.92 or less.

More specifically, the average number of -OH groups per molecule in the anhydrosugar alcohol composition may be 2.54 to 21.36, more specifically 2.60 to 21.30, and even more specifically 2.65 to 21.0. If the average number of -OH groups per molecule in the anhydrosugar alcohol composition is less than 2.54 or more than 21.36, the adhesive strength of the hot-melt adhesive using the polyurethane prepared by applying the polyol composition prepared using the same may be poor.

In one embodiment, the anhydrosugar alcohol composition of the present invention can be prepared by hydrogenating a glucose-containing saccharide composition (e.g., a saccharide composition comprising disaccharides or higher polysaccharides including glucose, mannose, fructose and maltose) to prepare a hydrogenated sugar composition, heating the obtained hydrogenated sugar composition under an acid catalyst to a dehydration reaction by heating and

conducting thin-film-distillation of the obtained dehydration reaction product. More specifically, the anhydrosugar alcohol composition of the present invention may be by-products remaining after obtaining a thin-film distillate by thin-film-distillation of the obtained dehydration reaction product.

More specifically, the hydrogenation may be carried out on a glucose-containing saccharide composition under a hydrogen pressure condition of 30 to 80 atm and a heating condition of 110°C to 135°C to prepare a hydrogenated sugar composition, and the dehydration reaction of the obtained hydrogenated sugar composition may be carried out under reduced pressure condition of 1 mmHg to 100 mmHg and heating condition of 105°C to 200°C to obtain a dehydration reaction product, and thin-film-distillation of the obtained dehydration reaction product may be conducted under reduced pressure condition of 2 mbar or less and heating condition of 150°C to 175°C, but the reaction conditions are not limited thereto.

The glucose content of the glucose-containing saccharide composition may be 41 wt% or more, 42 wt% or more, 45 wt% or more, 47 wt% or more, or 50 wt% or more, and may be 99.5 wt% or less, 99 wt% or less, 98.5 wt% or less, 98 wt% or less, 97.5 wt% or less, or 97 wt% or less-for example, 41 to 99.5 wt%, 45 to 98.5 wt%, or 50 to 98 wt%, based on the total weight of the glucose-containing saccharide composition.

If the glucose content in the saccharide composition is less than 41 wt%, the number average molecular weight, average number of -OH groups per molecule and polydispersity index of the anhydrosugar alcohol composition become too high, and the adhesive strength of the polyurethane hot-melt adhesive may be poor, and if the glucose content in the saccharide composition is more than 99.5 wt%, the number average molecular weight and polydispersity index of the polyol composition may be too low and the adhesive strength of the polyurethane hot-melt adhesive may be poor.

The content of polysaccharide alcohol (disaccharide or higher sugar alcohol) comprised in the hydrogenated sugar composition may be 0.8 wt% or more, 1 wt% or more, 2 wt% or more, or 3 wt% or more, and may be 57 wt% or less, 55 wt% or less, 52 wt% or less, 50 wt% or less, or 48 wt% or less-for example, 0.8 to 57 wt%, 1 to 55 wt% or 3 to 50 wt%, based on the total dry weight of the hydrogenated sugar composition (herein, the dry weight means the weight of solids remaining after water is removed from the hydrogenated sugar composition). If the content of the polysaccharide alcohol in the hydrogenated sugar composition is less than 0.8 wt%, the adhesive strength of the polyurethane hot-melt adhesive may be poor when a polyol composition is prepared using the hydrogenated sugar composition and a polyurethane hot-melt adhesive is prepared by applying the polyol composition. If the content of the polysaccharide alcohol in the hydrogenated sugar composition is more than 57 wt%, the viscosity of the polyol composition prepared using this hydrogenated sugar composition is very high, and the processability of the polyurethane hot-melt adhesive may be poor. In addition, the prepared polyurethane hot-melt adhesive has a lot of polyol groups, and as an excessive amount of isocyanate reacted therewith is used, the hardening property becomes stronger, resulting in poor adhesive strength.

In another aspect, the present invention provides a method for preparing a polyol composition comprising the step of performing an addition reaction of an anhydrosugar alcohol composition and an alkylene oxide, wherein more than 50 parts by weight and less than 4,000 parts by weight of alkylene oxide is reacted per 100 parts by weight of the anhydrosugar alcohol composition in the addition reaction, and the anhydrosugar alcohol composition comprises a) monoanhydrosugar alcohol; b) dianhydrosugar alcohol; c) polysaccharide alcohol represented by the above Formula 1; d) anhydrosugar alcohol derived from the polysaccharide alcohol represented by the above Formula 1; and e) a polymer of one or more of a) to d).

In the method for preparing the polyol composition of the present invention, descriptions of the anhydrosugar alcohol composition and the alkylene oxide are as described above.

In another aspect, the present invention provides a polyurethane prepolymer prepared by a reaction of the polyol composition of the present invention with a polyisocyanate.

In another aspect, the present invention provides a chain-extended polyurethane prepared by a reaction of the polyurethane prepolymer of the present invention with a chain extender.

In addition, in another aspect, the present invention provides a method for preparing a chain-extended polyurethane comprising (1) preparing a polyurethane prepolymer by reacting the polyol composition of the present invention with a polyisocyanate; and (2) reacting the polyurethane prepolymer with a chain extender.

In the method for preparing the chain-extended polyurethane of the present invention, the polyurethane prepolymer can be obtained by reacting the polyol composition with polyisocyanate-for example, a polyurethane prepolymer may be prepared by introducing the polyol composition sufficiently vacuum-dried in a four-necked reactor at 50 to 100°C, preferably 70 to 90°C, for 12 to 36 hours, preferably 20 to 28 hours and polyisocyanate, and reacting the mixture for 0.1 to 5 hours, preferably 0.5 to 2 hours while maintaining a temperature of 50 to 100°C, preferably 50 to 70°C under a nitrogen atmosphere.

The polyisocyanate compound usable in the present invention is not particularly limited, but specifically may be aromatic polyisocyanate compounds such as 1,3-phenylene diisocyanate, 1,4-phenylene diisocyanate, 2,4-tolylene diisocyanate (TDI), 2,6-tolylene diisocyanate, 4,4'-methylenediphenyl diisocyanate (MDI), 2,4-methylenediphenyl diisocyanate, 4,4'-diisocyanato biphenyl, 3,3'-dimethyl-4,4'-diisocyanato biphenyl, 3,3'-dimethyl-4,4'-diisocyanato diphenylmethane, 1,5-naphthylene diisocyanate, 4,4',4"-triphenylmethane triisocyanate, m-isocyanatophenylsulfonyl isocyanate

and p-isocyanato phenylsulfonyl isocyanate; aliphatic polyisocyanate compounds such as ethylene diisocyanate, tetramethylene diisocyanate, hexamethylene diisocyanate (HDI), dodecamethylene diisocyanate, 1,6,11-undecan triisocyanate, 2,2,4-trimethylhexamethylene diisocyanate, lysine diisocyanate, 2,6-diisocyanato methylcaproate, bis(2-isocyanatoethyl)fumarate, bis(2-isocyanatoethyl)carbonate, 2-isocyanatoethyl-2,6-diisocyanato hexanoate; alicyclic polyisocyanate compounds such as isophorone diisocyanate (IPDI), 4,4'-dicyclohexylmethane diisocyanate (hydrogenated MDI), cyclohexylene diisocyanate, methylcyclohexylene diisocyanate (hydrogenated TDI), bis(2-isocyanatoethyl)-4-cyclohexene-1,2-dicarboxylate, 2,5-norbornene diisocyanate and 2,6-norbornene diisocyanate, etc. These polyisocyanate compounds may be used alone or in combination of two or more. The chain extender used in the method for preparing the chain-extended polyurethane of the present invention is not particularly limited, and any conventional chain extender used in the production of polyurethane may be used without limitation. For example, the chain extender which is one selected from the group consisting of 1,4-butanediol, isosorbide, hydrazine monohydrate, ethylene diamine, dimethyl hydrazine, 1,6-hexamethylene bishydrazine, hexamethylene diamine, isophorone diamine, diaminophenylmethane or combinations thereof may be used, but the chain extenders are not limited thereto.

In the method for preparing the chain-extended polyurethane of the present invention, after adding the chain extender to the polyurethane prepolymer, by putting the mixture into the coated mold and curing at 80 to 200°C, preferably 100 to 150°C for 10 hours to 30 hours, preferably for 15 to 25 hours, the chain-extended polyurethane can be prepared.

In another aspect, the present invention provides a hot-melt adhesive comprising the chain-extended polyurethane of the present invention. The chain-extended polyurethane according to the present invention melts appropriately at an appropriate temperature (e.g., 180°C) and can be used for hot-melt adhesive applications.

The hot-melt adhesive of the present invention may additionally include additives commonly used in hot-melt adhesives.

The present invention is explained in more detail through the following Examples and Comparative Examples. However, the scope of the present invention is not limited thereby in any manner.

## [EXAMPLES]

### <Preparation of anhydrosugar alcohol composition >

<u>Preparation Example 1: Preparation of a polyol composition using 97 wt% glucose and a thin-film distiller</u>

1,819 g of a liquid hydrogenated sugar composition having a concentration of 55 wt% (based on solid content, sorbitol 96 wt%, mannitol 0.9 wt% and disaccharide or higher polysaccharide alcohol 3.1 wt%) was obtained by hydrogenating a glucose product having a purity of 97% in the presence of a nickel catalyst and under a temperature of 125°C and a hydrogen pressure of 60 atm. 1,000 g of a concentrated hydrogenated sugar composition was obtained by putting this composition in a batch reactor equipped with an agitator and heating it to 100°C for concentration.

The reactor was charged with 1,000 g of the concentrated hydrogenated sugar composition and 9.6 g of sulfuric acid. Thereafter, the temperature inside the reactor was raised to about 135°C, and a dehydration reaction was performed under a reduced pressure of about 45 mmHg to convert the concentrated hydrogenated sugar composition to anhydrosugar alcohol. After completion of the dehydration reaction, the temperature of the reaction product was cooled to 110°C or less, and about 15.7 g of 50% sodium hydroxide aqueous solution was added to neutralize the reaction product. Thereafter, the temperature was cooled to 100°C or less and the solution was concentrated for 1 hour or more under a reduced pressure of 45 mmHg to remove residual moisture and low-boiling substances to obtain about 831 g of the converted anhydrosugar alcohol solution. As a result of analyzing the obtained converted anhydrosugar alcohol solution by gas chromatography, the amount converted to isosorbide was 71.9 wt%, and using this, the molar conversion rate from sorbitol to isosorbide was calculated as 77.6%.

831 g of the obtained converted anhydrosugar alcohol solution was put into a thin-film distiller (SPD) to proceed with distillation. At this time, distillation was carried out at a temperature of 160°C and a vacuum pressure of 1 mbar, and about 589 g of distillate was obtained (distillation yield: about 70.9%). At this time, the purity of isosorbide in the distillate was measured to be 96.8%, and the distillation yield of isosorbide calculated therefrom was 95.3%. After separating the distillate, about 242 g of an anhydrosugar alcohol composition comprising 11.5 wt% of isosorbide (dianhydrosugar alcohol), 0.4 wt% of isomannide (dianhydrosugar alcohol), 7.4 wt% of sorbitan (monoanhydrosugar alcohol), 2.5 wt% of disaccharide or higher polysaccharide alcohols and anhydrosugar alcohol derived therefrom and 78.2 wt% of polymers thereof, and having the number average molecular weight of 208 g/mol, the polydispersity index of 1.25, the hydroxyl value of 751 mg KOH/g and an average number of -OH groups per molecule of 2.78 was obtained.

Preparation Example 2: Preparation of a polyol composition using a saccharide composition containing 85.2 wt% of glucose and a thin-film distiller

Except for the use of a 85.2 wt% glucose-containing saccharide composition (85.2 wt% of glucose and 14.8 wt% of total of mannose, fructose and polysaccharides (disaccharide or higher sugars such as maltose)) instead of a glucose product with a purity of 97%, the hydrogenation reaction was carried out in the same manner as in Example 1 to obtain 1,852 g of a liquid hydrogenated sugar composition having a concentration of 54 wt% (based on solid content, 84.1 wt% of sorbitol, 2.8 wt% of mannitol and 13.1 wt% of disaccharide or higher polysaccharide alcohol). 1,000 g of a concentrated hydrogenated sugar composition was obtained by putting this composition in a batch reactor equipped with an agitator and heating it to 100°C for concentration.

Except for changing the content of sulfuric acid from 9.6 g to 8.4 g and changing the content of 50% sodium hydroxide aqueous solution from 15.7 g to 13.7 g, 1,000 g of the concentrated hydrogenated sugar composition was converted into anhydrosugar alcohol by performing a dehydration reaction in the same manner as in Example 1. As a result of the dehydration reaction, about 846 g of the converted anhydrosugar alcohol solution was obtained. As a result of analyzing the obtained anhydrosugar alcohol solution by gas chromatography, the amount converted to isosorbide was 61.7 wt%, and using this, the molar conversion rate from sorbitol to isosorbide was calculated as 77.4%.

Thin-film distillation was performed on 846 g of the obtained converted anhydrosugar alcohol solution in the same manner as in Example 1 to obtain about 528 g of a distillate (distillation yield: about 62.4%). At this time, the purity of isosorbide in the distillate was measured to be 96.5%, and the distillation yield of isosorbide calculated therefrom was 97.6%. After separating the distillate, about 318 g of an anhydrosugar alcohol composition comprising 4.0 wt% of isosorbide (dianhydrosugar alcohol), 1.6 wt% of isomannide (dianhydrosugar alcohol), 2.1 wt% of sorbitan (monoanhydrosugar alcohol), 5.1 wt% of disaccharide or higher polysaccharide alcohols and anhydrosugar alcohol derived therefrom and 87.2 wt% of polymers thereof, and having the number average molecular weight of 720 g/mol, the polydispersity index of 2.54, the hydroxyl value of 754 mg KOH/g and an average number of -OH groups per molecule of 9.68 was obtained.

Preparation Example 3: Preparation of a polyol composition using a saccharide composition containing 50.2 wt% of glucose and a thin-film distiller

Except for the use of a 50.2 wt% glucose-containing saccharide composition (50.2 wt% of glucose and 49.8 wt% of total of mannose, fructose and polysaccharides (disaccharide or higher sugars such as maltose)) instead of a glucose product with a purity of 97%, the hydrogenation reaction was carried out in the same manner as in Example 1 to obtain 1,819 g of a liquid hydrogenated sugar composition having a concentration of 55 wt% (based on solid content, 48.5 wt% of sorbitol, 3.6 wt% of mannitol and 47.9 wt% of disaccharide or higher polysaccharide alcohol). 1,000 g of a concentrated hydrogenated sugar composition was obtained by putting this composition in a batch reactor equipped with an agitator and heating it to 100°C for concentration.

Except for changing the content of sulfuric acid from 9.6 g to 4.85 g and changing the content of 50% sodium hydroxide aqueous solution from 15.7 g to 7.9 g, 1,000 g of the concentrated hydrogenated sugar composition was converted into anhydrosugar alcohol by performing a dehydration reaction in the same manner as in Example 1. As a result of the dehydration reaction, about 890 g of the converted anhydrosugar alcohol solution was obtained. As a result of analyzing the obtained converted anhydrosugar alcohol solution by gas chromatography, the amount converted to isosorbide was 33.7 wt%, and using this, the molar conversion rate from sorbitol to isosorbide was calculated as 77.1%.

Thin-film distillation was performed on 890 g of the obtained converted anhydrosugar alcohol solution in the same manner as in Example 1 to obtain about 304 g of a distillate (distillation yield: about 34.2%). At this time, the purity of isosorbide in the distillate was measured to be 96.9%, and the distillation yield of isosorbide calculated therefrom was 98.3%. After separating the distillate, about 586 g of an anhydrosugar alcohol composition comprising 0.9 wt% of isosorbide (dianhydrosugar alcohol), 2.1 wt% of isomannide (dianhydrosugar alcohol), 0.9 wt% of sorbitan (monoanhydrosugar alcohol), 6.2 wt% of disaccharide or higher polysaccharide alcohols and anhydrosugar alcohol derived therefrom and 89.9 wt% of polymers thereof, and having the number average molecular weight of 1,480 g/mol, the polydispersity index of 3.19, the hydroxyl value of 755 mg KOH/g and an average number of -OH groups per molecule of 19.92 was obtained.

**<Preparation of alkylene oxide-added polyol composition>**

Example A1: Polyol composition in which 100 parts by weight of ethylene oxide was added per 100 parts by weight of the anhydrosugar alcohol composition of Preparation Example 1

100 parts by weight (100 g) of the anhydrosugar alcohol composition of Preparation Example 1 and 0.3 g of

KOH were put into a pressurized reactor, and pressurization and evacuation with nitrogen were repeated three times. Thereafter, the internal temperature of the reactor was raised to 100°C to remove moisture, and after all the moisture was removed, 100 parts by weight (100 g) of ethylene oxide was slowly injected and an addition reaction was performed at 100 to 140°C. Then, 4 g of metal adsorbent (Ambosol MP20) was added to remove metals and by-products, and stirring was performed for 1 to 5 hours while maintaining the internal temperature of the reactor at 100 to 120°C. After monitoring the residual metal content, the temperature inside the reactor was cooled to 60 to 90°C when the metal was completely removed and not detected, and then the mixture was filtered. Then, a polyol composition was obtained by purifying the filtrate using an ion exchange resin (UPRM 200, Samyang Corporation).

Example A2: Polyol composition in which 1,000 parts by weight of ethylene oxide was added per 100 parts by weight of the anhydrosugar alcohol composition of Preparation Example 1

A polyol composition was obtained in the same manner as in Example A1, except that the amount of the added ethylene oxide was changed from 100 parts by weight (100 g) to 1,000 parts by weight (1,000 g).

Example A3: Polyol composition in which 3,000 parts by weight of ethylene oxide was added per 100 parts by weight of the anhydrosugar alcohol composition of Preparation Example 1

A polyol composition was obtained in the same manner as in Example A1, except that the amount of the added ethylene oxide was changed from 100 parts by weight (100 g) to 3,000 parts by weight (3,000 g).

Example A4: Polyol composition in which 100 parts by weight of propylene oxide was added per 100 parts by weight of the anhydrosugar alcohol composition of Preparation Example 1

A polyol composition was obtained in the same manner as in Example A1, except that 100 parts by weight (100 g) of propylene oxide was used instead of ethylene oxide.

Example A5: Polyol composition in which 1,000 parts by weight of propylene oxide was added per 100 parts by weight of the anhydrosugar alcohol composition of Preparation Example 1

A polyol composition was obtained in the same manner as in Example A1, except that 1,000 parts by weight (1,000 g) of propylene oxide was used instead of ethylene oxide.

Example A6: Polyol composition in which 3,000 parts by weight of propylene oxide was added per 100 parts by weight of the anhydrosugar alcohol composition of Preparation Example 1

A polyol composition was obtained in the same manner as in Example A1, except that 3,000 parts by weight (3,000 g) of propylene oxide was used instead of ethylene oxide.

Example A7: Polyol composition in which 100 parts by weight of ethylene oxide was added per 100 parts by weight of the anhydrosugar alcohol composition of Preparation Example 2

A polyol composition was obtained in the same manner as in Example A1, except that 100 parts by weight (100 g) of the anhydrosugar alcohol composition of Preparation Example 2 was used instead of the anhydrosugar alcohol composition of Preparation Example 1.

Example A8: Polyol composition in which 1,000 parts by weight of ethylene oxide was added per 100 parts by weight of the anhydrosugar alcohol composition of Preparation Example 2

A polyol composition was obtained in the same manner as in Example A1, except that 100 parts by weight (100 g) of the anhydrosugar alcohol composition of Preparation Example 2 was used instead of the anhydrosugar alcohol composition of Preparation Example 1 and the amount of the added ethylene oxide was changed from 100 parts by weight (100 g) to 1,000 parts by weight (1,000 g).

Example A9: Polyol composition in which 3,000 parts by weight of ethylene oxide was added per 100 parts by weight of the anhydrosugar alcohol composition of Preparation Example 2

A polyol composition was obtained in the same manner as in Example A1, except that 100 parts by weight

(100 g) of the anhydrosugar alcohol composition of Preparation Example 2 was used instead of the anhydrosugar alcohol composition of Preparation Example 1 and the amount of the added ethylene oxide was changed from 100 parts by weight (100 g) to 3,000 parts by weight (3,000 g).

Example A10: Polyol composition in which 100 parts by weight of propylene oxide was added per 100 parts by weight of the anhydrosugar alcohol composition of Preparation Example 2

A polyol composition was obtained in the same manner as in Example A1, except that 100 parts by weight (100 g) of the anhydrosugar alcohol composition of Preparation Example 2 was used instead of the anhydrosugar alcohol composition of Preparation Example 1 and 100 parts by weight (100 g) of propylene oxide was used instead of ethylene oxide.

Example A11: Polyol composition in which 1,000 parts by weight of propylene oxide was added per 100 parts by weight of the anhydrosugar alcohol composition of Preparation Example 2

A polyol composition was obtained in the same manner as in Example A1, except that 100 parts by weight (100 g) of the anhydrosugar alcohol composition of Preparation Example 2 was used instead of the anhydrosugar alcohol composition of Preparation Example 1 and 1,000 parts by weight (1,000 g) of propylene oxide was used instead of ethylene oxide.

Example A12: Polyol composition in which 3,000 parts by weight of propylene oxide was added per 100 parts by weight of the anhydrosugar alcohol composition of Preparation Example 2

A polyol composition was obtained in the same manner as in Example A1, except that 100 parts by weight (100 g) of the anhydrosugar alcohol composition of Preparation Example 2 was used instead of the anhydrosugar alcohol composition of Preparation Example 1 and 3,000 parts by weight (3,000 g) of propylene oxide was used instead of ethylene oxide.

Example A13: Polyol composition in which 100 parts by weight of ethylene oxide was added per 100 parts by weight of the anhydrosugar alcohol composition of Preparation Example 3

A polyol composition was obtained in the same manner as in Example A1, except that 100 parts by weight (100 g) of the anhydrosugar alcohol composition of Preparation Example 3 was used instead of the anhydrosugar alcohol composition of Preparation Example 1.

Example A14: Polyol composition in which 1,000 parts by weight of ethylene oxide was added per 100 parts by weight of the anhydrosugar alcohol composition of Preparation Example 3

A polyol composition was obtained in the same manner as in Example A1, except that 100 parts by weight (100 g) of the anhydrosugar alcohol composition of Preparation Example 3 was used instead of the anhydrosugar alcohol composition of Preparation Example 1 and the amount of the added ethylene oxide was changed from 100 parts by weight (100 g) to 1,000 parts by weight (1,000 g).

Example A15: Polyol composition in which 3,000 parts by weight of ethylene oxide was added per 100 parts by weight of the anhydrosugar alcohol composition of Preparation Example 3

A polyol composition was obtained in the same manner as in Example A1, except that 100 parts by weight (100 g) of the anhydrosugar alcohol composition of Preparation Example 3 was used instead of the anhydrosugar alcohol composition of Preparation Example 1 and the amount of the added ethylene oxide was changed from 100 parts by weight (100 g) to 3,000 parts by weight (3,000 g).

Example A16: Polyol composition in which 100 parts by weight of propylene oxide was added per 100 parts by weight of the anhydrosugar alcohol composition of Preparation Example 3

A polyol composition was obtained in the same manner as in Example A1, except that 100 parts by weight (100 g) of the anhydrosugar alcohol composition of Preparation Example 3 was used instead of the anhydrosugar alcohol composition of Preparation Example 1 and 100 parts by weight (100 g) of propylene oxide was used instead of ethylene oxide.

Example A17: Polyol composition in which 1,000 parts by weight of propylene oxide was added per 100 parts by weight of the anhydrosugar alcohol composition of Preparation Example 3

A polyol composition was obtained in the same manner as in Example A1, except that 100 parts by weight (100 g) of the anhydrosugar alcohol composition of Preparation Example 3 was used instead of the anhydrosugar alcohol composition of Preparation Example 1 and 1,000 parts by weight (1,000 g) of propylene oxide was used instead of ethylene oxide.

Example A18: Polyol composition in which 3,000 parts by weight of propylene oxide was added per 100 parts by weight of the anhydrosugar alcohol composition of Preparation Example 3

A polyol composition was obtained in the same manner as in Example A1, except that 100 parts by weight (100 g) of the anhydrosugar alcohol composition of Preparation Example 3 was used instead of the anhydrosugar alcohol composition of Preparation Example 1 and 3,000 parts by weight (3,000 g) of propylene oxide was used instead of ethylene oxide.

Example A19: A polyol composition obtained by adding 50 parts by weight of ethylene oxide and then adding 50 parts by weight of propylene oxide per 100 parts by weight of the anhydrosugar alcohol composition of Preparation Example 1

100 parts by weight (100 g) of the anhydrosugar alcohol composition of Preparation Example 1 and 0.3 g of KOH were put into a pressurized reactor, and pressurization and evacuation with nitrogen were repeated three times. Thereafter, the internal temperature of the reactor was raised to 100°C to remove moisture, and after all the moisture was removed, 50 parts by weight (50 g) of ethylene oxide was slowly injected and an addition reaction was performed at 100 to 140°C. Then, 50 parts by weight (50 g) of propylene oxide was slowly injected and an addition reaction was performed at 100 to 140°C. Then, 4 g of metal adsorbent (Ambosol MP20) was added to remove metals and by-products, and stirring was performed for 1 to 5 hours while maintaining the internal temperature of the reactor at 100 to 120°C. After monitoring the residual metal content, the temperature inside the reactor was cooled to 60 to 90°C when the metal was completely removed and not detected, and then the mixture was filtered. Then, a polyol composition was obtained by purifying the filtrate using an ion exchange resin (UPRM 200, Samyang Corporation).

Example A20: A polyol composition obtained by adding 500 parts by weight of ethylene oxide and then adding 500 parts by weight of propylene oxide per 100 parts by weight of the anhydrosugar alcohol composition of Preparation Example 1

A polyol composition was obtained in the same manner as in Example A19, except that the amount of ethylene oxide was changed from 50 parts by weight (50 g) to 500 parts by weight (500 g) and the amount of propylene oxide was changed from 50 parts by weight (50 g) to 500 parts by weight (500 g).

Example A21: A polyol composition obtained by adding 500 parts by weight of propylene oxide and then adding 500 parts by weight of ethylene oxide per 100 parts by weight of the anhydrosugar alcohol composition of Preparation Example 1

A polyol composition was obtained in the same manner as in Example A19, except that first an addition reaction of 100 parts by weight (100 g) of the anhydrosugar alcohol composition and 500 parts by weight (500 g) of propylene oxide performed, and then an addition reaction of 500 parts by weight (500 g) of ethylene oxide was performed.

Example A22: A polyol composition obtained by adding 1,500 parts by weight of propylene oxide and then adding 1,500 parts by weight of ethylene oxide per 100 parts by weight of the anhydrosugar alcohol composition of Preparation Example 1

A polyol composition was obtained in the same manner as in Example A19, except that first an addition reaction of 100 parts by weight (100 g) of the anhydrosugar alcohol composition and 1,500 parts by weight (1,500 g) of propylene oxide performed, and then an addition reaction of 1,500 parts by weight (1,500 g) of ethylene oxide was performed.

Comparative Example A1: A polyol composition in which 50 parts by weight of ethylene oxide was added per 100 parts by weight of the anhydrosugar alcohol composition of Preparation Example 1

A polyol composition was obtained in the same manner as in Example A1, except that the amount of the added ethylene oxide was changed from 100 parts by weight (100 g) to 50 parts by weight (50 g).

Comparative Example A2: Polyol composition in which 4,000 parts by weight of ethylene oxide was added per 100 parts by weight of the anhydrosugar alcohol composition of Preparation Example 1

A polyol composition was obtained in the same manner as in Example A1, except that the amount of the added ethylene oxide was changed from 100 parts by weight (100 g) to 4,000 parts by weight (4,000 g).

Comparative Example A3: A polyol composition obtained by adding 50 parts by weight of propylene oxide per 100 parts by weight of the anhydrosugar alcohol composition of Preparation Example 1

A polyol composition was obtained in the same manner as in Example A1, except that 50 parts by weight (50 g) of propylene oxide was used instead of ethylene oxide.

Comparative Example A4: A polyol composition in which 4,000 parts by weight of propylene oxide was added per 100 parts by weight of the anhydrosugar alcohol composition of Preparation Example 1

A polyol composition was obtained in the same manner as in Example A1, except that 4,000 parts by weight (4,000 g) of propylene oxide was used instead of ethylene oxide.

Comparative Example A5: A polyol composition in which 50 parts by weight of ethylene oxide was added per 100 parts by weight of the anhydrosugar alcohol composition of Preparation Example 2

A polyol composition was obtained in the same manner as in Example A1 except that 100 parts by weight (100 g) of the anhydrosugar alcohol composition of Preparation Example 2 was used instead of the anhydrosugar alcohol composition of Preparation Example 1 and the content of the added ethylene oxide was changed from 100 parts by weight (100 g) to 50 parts by weight (50 g)

Comparative Example A6: A polyol composition in which 4,000 parts by weight of ethylene oxide was added per 100 parts by weight of the anhydrosugar alcohol composition of Preparation Example 2

A polyol composition was obtained in the same manner as in Example A1 except that 100 parts by weight (100 g) of the anhydrosugar alcohol composition of Preparation Example 2 was used instead of the anhydrosugar alcohol composition of Preparation Example 1 and the content of the added ethylene oxide was changed from 100 parts by weight (100 g) to 4,000 parts by weight (4,000 g)

Comparative Example A7: A polyol composition in which 50 parts by weight of propylene oxide was added per 100 parts by weight of the anhydrosugar alcohol composition of Preparation Example 2

A polyol composition was obtained in the same manner as in Example A1 except that 100 parts by weight (100 g) of the anhydrosugar alcohol composition of Preparation Example 2 was used instead of the anhydrosugar alcohol composition of Preparation Example 1 and 50 parts by weight (50 g) of propylene oxide was used instead of ethylene oxide,

Comparative Example A8: A polyol composition in which 4,000 parts by weight of propylene oxide was added per 100 parts by weight of the anhydrosugar alcohol composition of Preparation Example 2

A polyol composition was obtained in the same manner as in Example A1 except that 100 parts by weight (100 g) of the anhydrosugar alcohol composition of Preparation Example 2 instead of the anhydrosugar alcohol composition of Preparation Example 1 and 4,000 parts by weight (4,000 g) of propylene oxide was used instead of ethylene oxide.

Comparative Example A9: A polyol composition in which 50 parts by weight of ethylene oxide was added per 100 parts by weight of the anhydrosugar alcohol composition of Preparation Example 3

A polyol composition was obtained in the same manner as in Example A1 except that 100 parts by weight (100 g) of the anhydrosugar alcohol composition of Preparation Example 3 was used instead of the anhydrosugar alcohol composition of Preparation Example 1 and the content of the added ethylene oxide was changed from 100 parts by weight (100 g) to 50 parts by weight (50 g).

Comparative Example A10: A polyol composition in which 4,000 parts by weight of ethylene oxide was added per 100 parts by weight of the anhydrosugar alcohol composition of Preparation Example 3

A polyol composition was obtained in the same manner as in Example A1 except that 100 parts by weight (100 g) of the anhydrosugar alcohol composition of Preparation Example 3 was used instead of the anhydrosugar alcohol composition of Preparation Example 1 and the content of the added ethylene oxide was changed from 100 parts by weight (100 g) to 4,000 parts by weight (4,000 g).

Comparative Example A11: A polyol composition in which 50 parts by weight of propylene oxide was added per 100 parts by weight of the anhydrosugar alcohol composition of Preparation Example 3

A polyol composition was obtained in the same manner as in Example A1 except that 100 parts by weight (100 g) of the anhydrosugar alcohol composition of Preparation Example 3 instead of the anhydrosugar alcohol composition of Preparation Example 1 and 50 parts by weight (50 g) of propylene oxide was used instead of ethylene oxide.

Comparative Example A12: A polyol composition in which 4,000 parts by weight of propylene oxide was added per 100 parts by weight of the anhydrosugar alcohol composition of Preparation Example 3

A polyol composition was obtained in the same manner as in Example A1 except that 100 parts by weight (100 g) of the anhydrosugar alcohol composition of Preparation Example 3 was used instead of the anhydrosugar alcohol composition of Preparation Example 1 and 4,000 parts by weight (4,000 g) of propylene oxide was used instead of ethylene oxide.

**[Method for measuring yield of anhydrosugar alcohol composition]**

1) Molar conversion rate to isosorbide (ISB)

$$\text{Molar conversion rate to ISB (\%)} = \frac{\text{mole of generated ISB}}{\text{mole of used sorbitol}} \times 100\ (\%)$$

2) Isosorbide (ISB) conversion content

Using gas chromatography analysis, the content (wt%) of isosorbide in the converted anhydrosugar alcohol solution was measured, and the isosorbide conversion content indicates the purity of isosorbide (ISB) in the converted anhydrosugar alcohol solution.

3) Distillation yield

$$\text{Distillation yield (\%)} = \frac{\text{Mass of distillate (g)}}{\text{Mass of the converted anhydrosugar alcohol solution (g)}} \times 100\ (\%)$$

4) Distillation yield of isosorbide (ISB)

$$\text{Distillation yield of ISB} = \frac{\text{Mass of isosorbide in distillate (g)}}{\text{Mass of isosorbide in the converted anhydrosugar alcohol solution (g)}} \times 100\ (\%)$$

**[Method for measuring physical properties of anhydrosugar alcohol composition]**

1) Number average molecular weight (Mn) and polydispersity index (PDI)

After dissolving 1 to 3 parts by weight of each of the anhydrosugar alcohol compositions prepared in the above Preparation Examples in N,N-dimethylformamide, number average molecular weight (Mn) and polydispersity index (PDI) were measured using a Gel Permeation Chromatography (GPC) apparatus (Agilent Co.). The column used at this time was PLgel 3μm MIXED-E 300×7.5mm (Agilent Co.), and the column temperature was 50°C. The developing solvent used was N,N-dimethylformamide containing 0.05 M NaBr, which was used by flowing at 0.5 mL/min, and polystyrene (Aldrich Co.) was used as a standard material.

2) hydroxyl value

After esterification of each of the anhydrosugar alcohol compositions prepared in the Preparation Examples with an excess of phthalic anhydride under an imidazole catalyst according to ASTM D-4274D, the hydroxyl value of the anhydrosugar alcohol composition was measured by titrating the remaining phthalic anhydride with 0.5 N sodium hydroxide (NaOH).

3) Average number of -OH groups per molecule

The average number of -OH groups per molecule in the polyol composition was calculated according to the formula below.

**[Average number of -OH groups per molecule] = (hydroxyl value × number average molecular weight) / 56,100**

**<Preparation of polyurethane using alkylene oxide-added polyol composition>**

Example B1: Preparation of chain-extended polyurethane using the polyol composition of Example A1 as polyol and isosorbide as chain extender

100.00 g of the polyol composition of Example A1 which was sufficiently vacuum-dried at 80°C for 24 hours and 420.35 g of 4,4'-methylenediphenyl diisocyanate (MDI) were put into a 4-necked reactor, and then a polyurethane prepolymer was prepared by reacting for 1 hour while maintaining a temperature of 60°C under a nitrogen atmosphere. Subsequently, when the measured NCO% of the polyurethane prepolymer reached the theoretical NCO%, 61.37 g of isosorbide was added as a chain extender and mixed. The mixture was put into a silicone-coated mold and cured at 110°C for 16 hours to prepare a chain-extended polyurethane.

Example B2: Preparation of chain-extended polyurethane using the polyol composition of Example A2 as polyol and isosorbide as chain extender

A chain-extended polyurethane was prepared in the same manner as in Example B1, except that 100.00 g of the polyol composition of Example A2 was used instead of the polyol composition of Example A1, the content of 4,4'-methylenediphenyl diisocyanate (MDI) was changed from 420.35 g to 95.50 g and the content of isosorbide was changed from 61.37 g to 13.94 g.

Example B3: Preparation of chain-extended polyurethane using the polyol composition of Example A3 as polyol and isosorbide as chain extender

A chain-extended polyurethane was prepared in the same manner as in Example B1, except that 100.00 g of the polyol composition of Example A3 was used instead of the polyol composition of Example A1, the content of 4,4'-methylenediphenyl diisocyanate (MDI) was changed from 420.35 g to 31.71 g and the content of isosorbide was changed from 61.37 g to 5.07 g.

Example B4: Preparation of chain-extended polyurethane using the polyol composition of Example A4 as polyol and isosorbide as chain extender

A chain-extended polyurethane was prepared in the same manner as in Example B1, except that 100.00 g of the polyol composition of Example A4 was used instead of the polyol composition of Example A1, the content of 4,4'-methylenediphenyl diisocyanate (MDI) was changed from 420.35 g to 395.80 g and the content of isosorbide was changed from 61.37 g to 57.78 g.

Example B5: Preparation of chain-extended polyurethane using the polyol composition of Example A5 as polyol and isosorbide as chain extender

A chain-extended polyurethane was prepared in the same manner as in Example B1, except that 100.00 g of the polyol composition of Example A5 was used instead of the polyol composition of Example A1, the content of 4,4'-methylenediphenyl diisocyanate (MDI) was changed from 420.35 g to 84.67 g and the content of isosorbide was changed from 61.37 g to 12.36 g.

Example B6: Preparation of chain-extended polyurethane using the polyol composition of Example A6 as polyol and isosorbide as chain extender

A chain-extended polyurethane was prepared in the same manner as in Example B1, except that 100.00 g of the polyol composition of Example A6 was used instead of the polyol composition of Example A1, the content of 4,4'-methylenediphenyl diisocyanate (MDI) was changed from 420.35 g to 30.82 g and the content of isosorbide was changed from 61.37 g to 4.50 g.

Example B7: Preparation of chain-extended polyurethane using the polyol composition of Example A7 as polyol and isosorbide as chain extender

A chain-extended polyurethane was prepared in the same manner as in Example B1, except that 100.00 g of the polyol composition of Example A7 was used instead of the polyol composition of Example A1, the content of 4,4'-methylenediphenyl diisocyanate (MDI) was changed from 420.35 g to 422.84 g and the content of isosorbide was changed from 61.37 g to 61.73 g.

Example B8: Preparation of chain-extended polyurethane using the polyol composition of Example A8 as polyol and isosorbide as chain extender

A chain-extended polyurethane was prepared in the same manner as in Example B1, except that 100.00 g of the polyol composition of Example A8 was used instead of the polyol composition of Example A1, the content of 4,4'-methylenediphenyl diisocyanate (MDI) was changed from 420.35 g to 96.07 g and the content of isosorbide was changed from 61.37 g to 14.03 g.

Example B9: Preparation of chain-extended polyurethane using the polyol composition of Example A9 as polyol and isosorbide as chain extender

A chain-extended polyurethane was prepared in the same manner as in Example B1, except that 100.00 g of the polyol composition of Example A9 was used instead of the polyol composition of Example A1, the content of 4,4'-methylenediphenyl diisocyanate (MDI) was changed from 420.35 g to 34.91 g and the content of isosorbide was changed from 61.37 g to 5.10 g.

Example B10: Preparation of chain-extended polyurethane using the polyol composition of Example A10 as polyol and isosorbide as chain extender

A chain-extended polyurethane was prepared in the same manner as in Example B1, except that 100.00 g of the polyol composition of Example A10 was used instead of the polyol composition of Example A1, the content of 4,4'-methylenediphenyl diisocyanate (MDI) was changed from 420.35 g to 398.14 g and the content of isosorbide was changed from 61.37 g to 58.13 g.

Example B11: Preparation of chain-extended polyurethane using the polyol composition of Example A11 as polyol and isosorbide as chain extender

A chain-extended polyurethane was prepared in the same manner as in Example B1, except that 100.00 g of the polyol composition of Example A11 was used instead of the polyol composition of Example A1, the content of 4,4'-methylenediphenyl diisocyanate (MDI) was changed from 420.35 g to 85.17 g and the content of isosorbide was changed from 61.37 g to 12.43 g.

Example B12: Preparation of chain-extended polyurethane using the polyol composition of Example A12 as polyol and isosorbide as chain extender

A chain-extended polyurethane was prepared in the same manner as in Example B1, except that 100.00 g of the polyol composition of Example A12 was used instead of the polyol composition of Example A1, the content of 4,4'-methylenediphenyl diisocyanate (MDI) was changed from 420.35 g to 31.01 g and the content of isosorbide was changed from 61.37 g to 4.50 g.

Example B13: Preparation of chain-extended polyurethane using the polyol composition of Example A13 as polyol and isosorbide as chain extender

A chain-extended polyurethane was prepared in the same manner as in Example B1, except that 100.00 g of the polyol composition of Example A13 was used instead of the polyol composition of Example A1, the content of 4,4'-methylenediphenyl diisocyanate (MDI) was changed from 420.35 g to 423.31 g and the content of isosorbide was changed from 61.37 g to 61.80 g.

Example B14: Preparation of chain-extended polyurethane using the polyol composition of Example A14 as polyol and isosorbide as chain extender

A chain-extended polyurethane was prepared in the same manner as in Example B1, except that 100.00 g of the polyol composition of Example A14 was used instead of the polyol composition of Example A1, the content of 4,4'-methylenediphenyl diisocyanate (MDI) was changed from 420.35 g to 96.17 g and the content of isosorbide was changed from 61.37 g to 14.04 g.

Example B15: Preparation of chain-extended polyurethane using the polyol composition of Example A15 as polyol and isosorbide as chain extender

A chain-extended polyurethane was prepared in the same manner as in Example B1, except that 100.00 g of the polyol composition of Example A15 was used instead of the polyol composition of Example A1, the content of 4,4'-methylenediphenyl diisocyanate (MDI) was changed from 420.35 g to 34.95 g and the content of isosorbide was changed from 61.37 g to 5.10 g.

Example B16: Preparation of chain-extended polyurethane using the polyol composition of Example A16 as polyol and isosorbide as chain extender

A chain-extended polyurethane was prepared in the same manner as in Example B1, except that 100.00 g of the polyol composition of Example A16 was used instead of the polyol composition of Example A1, the content of 4,4'-methylenediphenyl diisocyanate (MDI) was changed from 420.35 g to 398.59 g and the content of isosorbide was changed from 61.37 g to 58.19 g.

Example B17: Preparation of chain-extended polyurethane using the polyol composition of Example A17 as polyol and isosorbide as chain extender

A chain-extended polyurethane was prepared in the same manner as in Example B1, except that 100.00 g of the polyol composition of Example A17 was used instead of the polyol composition of Example A1, the content of 4,4'-methylenediphenyl diisocyanate (MDI) was changed from 420.35 g to 85.26 g and the content of isosorbide was changed from 61.37 g to 12.45 g.

Example B18: Preparation of chain-extended polyurethane using the polyol composition of Example A18 as polyol and isosorbide as chain extender

A chain-extended polyurethane was prepared in the same manner as in Example B1, except that 100.00 g of the polyol composition of Example A18 was used instead of the polyol composition of Example A1, the content of 4,4'-methylenediphenyl diisocyanate (MDI) was changed from 420.35 g to 31.04 g and the content of isosorbide was changed from 61.37 g to 4.53 g.

Example B19: Preparation of chain-extended polyurethane using the polyol composition of Example A19 as polyol and isosorbide as chain extender

A chain-extended polyurethane was prepared in the same manner as in Example B1, except that 100.00 g of the polyol composition of Example A19 was used instead of the polyol composition of Example A1, the content of 4,4'-methylenediphenyl diisocyanate (MDI) was changed from 420.35 g to 506.81 g and the content of isosorbide was changed from 61.37 g to 73.99 g.

Example B20: Preparation of chain-extended polyurethane using the polyol composition of Example A20 as polyol and isosorbide as chain extender

A chain-extended polyurethane was prepared in the same manner as in Example B1, except that 100.00 g of the polyol composition of Example A20 was used instead of the polyol composition of Example A1, the content of 4,4'-methylenediphenyl diisocyanate (MDI) was changed from 420.35 g to 158.73 g and the content of isosorbide was changed from 61.37 g to 23.17 g.

Example B21: Preparation of chain-extended polyurethane using the polyol composition of Example A21 as polyol and isosorbide as chain extender

A chain-extended polyurethane was prepared in the same manner as in Example B1, except that 100.00 g of the polyol composition of Example A21 was used instead of the polyol composition of Example A1, the content of 4,4'-methylenediphenyl diisocyanate (MDI) was changed from 420.35 g to 158.71 g and the content of isosorbide was changed from 61.37 g to 23.15 g.

Example B22: Preparation of chain-extended polyurethane using the polyol composition of Example A22 as polyol and isosorbide as chain extender

A chain-extended polyurethane was prepared in the same manner as in Example B1, except that 100.00 g of the polyol composition of Example A22 was used instead of the polyol composition of Example A1, the content of 4,4'-methylenediphenyl diisocyanate (MDI) was changed from 420.35 g to 62.46 g and the content of isosorbide was changed from 61.37 gto 9.12 g.

Comparative Example B1: Preparation of chain-extended polyurethane using the polyol composition of Comparative Example A1 as polyol and isosorbide as chain extender

A chain-extended polyurethane was prepared in the same manner as in Example B1, except that 100.00 g of the polyol composition of Comparative Example A1 was used instead of the polyol composition of Example A1, the content of 4,4'-methylenediphenyl diisocyanate (MDI) was changed from 420.35 g to 516.28 g and the content of isosorbide was changed from 61.37 g to 75.37 g.

Comparative Example B2: Preparation of chain-extended polyurethane using the polyol composition of Comparative Example A2 as polyol and isosorbide as chain extender

A chain-extended polyurethane was prepared in the same manner as in Example B1, except that 100.00 g of the polyol composition of Comparative Example A2 was used instead of the polyol composition of Example A1, the content of 4,4'-methylenediphenyl diisocyanate (MDI) was changed from 420.35 g to 26.03 g and the content of isosorbide was changed from 61.37 g to 3.80 g.

Comparative Example B3: Preparation of chain-extended polyurethane using the polyol composition of Comparative Example A3 as polyol and isosorbide as chain extender

A chain-extended polyurethane was prepared in the same manner as in Example B1, except that 100.00 g of the polyol composition of Comparative Example A3 was used instead of the polyol composition of Example A1, the content of 4,4'-methylenediphenyl diisocyanate (MDI) was changed from 420.35 g to 497.34 g and the content of isosorbide was changed from 61.37 g to 72.61 g.

Comparative Example B4: Preparation of chain-extended polyurethane using the polyol composition of Comparative Example A4 as polyol and isosorbide as chain extender

A chain-extended polyurethane was prepared in the same manner as in Example B1, except that 100.00 g of the polyol composition of Comparative Example A4 was used instead of the polyol composition of Example A1, the content of 4,4'-methylenediphenyl diisocyanate (MDI) was changed from 420.35 g to 23.39 g and the content of isosorbide was changed from 61.37 g to 3.41 g.

Comparative Example B5: Preparation of chain-extended polyurethane using the polyol composition of Comparative Example A5 as polyol and isosorbide as chain extender

A chain-extended polyurethane was prepared in the same manner as in Example B1, except that 100.00 g of the polyol composition of Comparative Example A5 was used instead of the polyol composition of Example A1, the content of 4,4'-methylenediphenyl diisocyanate (MDI) was changed from 420.35 g to 519.33 g and the content of isosorbide was changed from 61.37 g to 75.82 g.

Comparative Example B6: Preparation of chain-extended polyurethane using the polyol composition of Comparative Example A6 as polyol and isosorbide as chain extender

A chain-extended polyurethane was prepared in the same manner as in Example B1, except that 100.00 g of the polyol composition of Comparative Example A6 was used instead of the polyol composition of Example A1, the content of 4,4'-methylenediphenyl diisocyanate (MDI) was changed from 420.35 g to 26.19 g and the content of isosorbide was changed from 61.37 g to 3.82 g.

Comparative Example B7: Preparation of chain-extended polyurethane using the polyol composition of Comparative Example A7 as polyol and isosorbide as chain extender

A chain-extended polyurethane was prepared in the same manner as in Example B1, except that 100.00 g of the polyol composition of Comparative Example A7 was used instead of the polyol composition of Example A1, the content of 4,4'-methylenediphenyl diisocyanate (MDI) was changed from 420.35 g to 500.28 g and the content of isosorbide was changed from 61.37 g to 73.04 g.

Comparative Example B8: Preparation of chain-extended polyurethane using the polyol composition of Comparative Example A8 as polyol and isosorbide as chain extender

A chain-extended polyurethane was prepared in the same manner as in Example B1, except that 100.00 g of the polyol composition of Comparative Example A8 was used instead of the polyol composition of Example A1, the content of 4,4'-methylenediphenyl diisocyanate (MDI) was changed from 420.35 g to 23.53 g and the content of isosorbide was changed from 61.37 g to 3.43 g.

Comparative Example B9: Preparation of chain-extended polyurethane using the polyol composition of Comparative Example A9 as polyol and isosorbide as chain extender

A chain-extended polyurethane was prepared in the same manner as in Example B1, except that 100.00 g of the polyol composition of Comparative Example A9 was used instead of the polyol composition of Example A1, the content of 4,4'-methylenediphenyl diisocyanate (MDI) was changed from 420.35 g to 519.91 g and the content of isosorbide was changed from 61.37 g to 75.90 g.

Comparative Example B10: Preparation of chain-extended polyurethane using the polyol composition of Comparative Example A10 as polyol and isosorbide as chain extender

A chain-extended polyurethane was prepared in the same manner as in Example B1, except that 100.00 g of the polyol composition of Comparative Example A10 was used instead of the polyol composition of Example A1, the content of 4,4'-methylenediphenyl diisocyanate (MDI) was changed from 420.35 g to 26.21 g and the content of isosorbide was changed from 61.37 g to 3.83 g.

Comparative Example B11: Preparation of chain-extended polyurethane using the polyol composition of Comparative Example A11 as polyol and isosorbide as chain extender

A chain-extended polyurethane was prepared in the same manner as in Example B1, except that 100.00 g of the polyol composition of Comparative Example A11 was used instead of the polyol composition of Example A1, the content of 4,4'-methylenediphenyl diisocyanate (MDI) was changed from 420.35 g to 500.84 g and the content of isosorbide was changed from 61.37 g to 73.12 g.

Comparative Example B12: Preparation of chain-extended polyurethane using the polyol composition of Comparative Example A12 as polyol and isosorbide as chain extender

A chain-extended polyurethane was prepared in the same manner as in Example B1, except that 100.00 g of the polyol composition of Comparative Example A12 was used instead of the polyol composition of Example A1, the content of 4,4'-methylenediphenyl diisocyanate (MDI) was changed from 420.35 g to 23.55 g and the content of isosorbide was changed from 61.37 g to 3.44 g.

Comparative Example B13: Preparation of chain-extended polyurethane using PTMEG as polyol and isosorbide as chain extender

A chain-extended polyurethane was prepared in the same manner as in Example B1, except that 100.00 g of commercially available polytetramethylene ether glycol (PTMEG, weight average molecular weight 1,000) was used instead of the polyol composition of Example A1, the content of 4,4'-methylenediphenyl diisocyanate (MDI) was changed from 420.35 g to 50.05 g and the content of isosorbide was changed from 61.37 g to 14.61 g.

**<Preparation of hot-melt specimen>**

Each of the chain-extended polyurethanes prepared in Examples B1 to B22 and Comparative Examples B1 to B13 was applied to two stainless steels (20 mm × 100 mm) in a uniform size (20 mm × 20 mm), and then using a hot press, a pressure of 1 MPa was applied at a temperature of 180°C for 10 minutes to prepare a specimen for measuring adhesive strength. The adhesive strength of the specimen was measured as follows, and the results are shown in Table 1 below.

[Method of measuring property]

(1) Adhesive strength

The measurement was performed at a speed of 5 mm/min using UTM (Instron, Instron 5967). Specifically, the adhesive strength was measured a total of 5 times for each hot-melt specimen, and the average value was calculated.

[Table 1]

| Categories | | Components | | | Property |
|---|---|---|---|---|---|
| | | Polyol | Isocyanate | Chain extender | Adhesive strength (MPa) |
| Examples | B1 | Example A1 | MDI | Isosorbide | 5.1 |
| | B2 | Example A2 | | | 4.5 |
| | B3 | Example A3 | | | 3.2 |
| | B4 | Example A4 | | | 6.2 |
| | B5 | Example A5 | | | 5.6 |
| | B6 | Example A6 | | | 3.1 |
| | B7 | Example A7 | | | 8.2 |
| | B8 | Example A8 | | | 5.3 |
| | B9 | Example A9 | | | 4.2 |
| | B10 | Example A10 | | | 7.5 |
| | B11 | Example A11 | | | 5.5 |
| | B12 | Example A12 | | | 4.1 |
| | B13 | Example A13 | | | 11.1 |
| | B14 | Example A14 | | | 8.5 |
| | B15 | Example A15 | | | 6.4 |
| | B16 | Example A16 | | | 11.3 |
| | B17 | Example A17 | | | 8.6 |
| | B18 | Example A18 | | | 6.5 |
| | B19 | Example A19 | | | 5.6 |

(continued)

| Categories | | Components | | | Property |
|---|---|---|---|---|---|
| | | Polyol | Isocyanate | Chain extender | Adhesive strength (MPa) |
| | B20 | Example A20 | | | 5.1 |
| | B21 | Example A21 | | | 5.3 |
| | B22 | Example A22 | | | 3.2 |
| Comparative Example | B1 | Comparative Example A1 | | | Cohesive peeling |
| | B2 | Comparative Example A2 | | | Surface peeling |
| | B3 | Comparative Example A3 | | | Cohesive peeling |
| | B4 | Comparative Example A4 | | | Surface peeling |
| | B5 | Comparative Example A5 | | | Cohesive peeling |
| | B6 | Comparative Example A6 | | | Surface peeling |
| | B7 | Comparative Example A7 | | | Cohesive peeling |
| | B8 | Comparative Example A8 | | | Surface peeling |
| | B9 | Comparative Example A9 | | | Cohesive peeling |
| | B10 | Comparative Example A10 | | | Surface peeling |
| | B11 | Comparative Example A11 | | | Cohesive peeling |
| | B12 | Comparative Example A12 | | | Surface peeling |
| | B13 | PTMEG | | | Flow down |

As described in Table 2 above, it was confirmed that the hot-melt specimens of Examples B1 to B22 according to the present invention exhibited excellent adhesive strength and economic feasibility was improved due to cost reduction.

However, in the case of the hot-melt specimens of Comparative Examples B1, B3, B5, B7, B9 and B11, it was confirmed that the soft portion that imparts flexibility in the hot-melt specimen is too small, so that cohesive peeling (referring to the case where the hot-melt adhesive itself is broken) occurs. In the case of the hot-melt specimens of Comparative Examples B2, B4, B6, B8, B10 and B12, it was confirmed that the hard portion that imparts adhesive strength in the hot-melt specimen is too small, so that surface peeling (referring to the case where the adhesive interface is peeled off) occurs.

On the other hand, the hot-melt specimen of Comparative Example B13 using PTMEG, a conventionally commercialized polyol, melted too much at a temperature of 180°C and flowed down, making it impossible to measure because the adhesion of the two stainless steels was not uniform.

## Claims

**1.** A polyol composition prepared by addition reaction of 100 parts by weight of an anhydrosugar alcohol composition

and more than 50 parts by weight to less than 4,000 parts by weight of an alkylene oxide, wherein the anhydrosugar alcohol composition comprises a) monoanhydrosugar alcohol; b) dianhydrosugar alcohol; c) polysaccharide alcohol represented by the following Formula 1; d) anhydrosugar alcohol derived from the polysaccharide alcohol represented by the following Formula 1; and e) a polymer of one or more of a) to d):

[Formula 1]

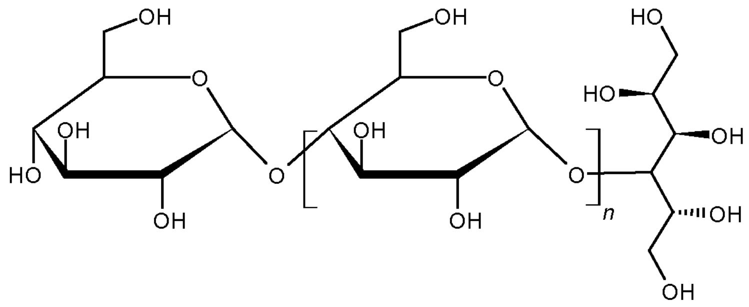

In Formula 1, n is an integer of 0 to 4.

2. The polyol composition according to claim 1, wherein the anhydrosugar alcohol composition satisfies the following i) to iii):

(i) the anhydrosugar alcohol composition has a number average molecular weight (Mn) of 193 to 1,589 g/mol;
(ii) the anhydrosugar alcohol composition has a polydispersity index (PDI) of 1.13 to 3.41; and
(iii) the average number of -OH groups per molecule in the anhydrosugar alcohol composition is 2.54 to 21.36.

3. The polyol composition according to claim 1, wherein d) anhydrosugar alcohol derived from the polysaccharide alcohol represented by Formula 1 is selected from a compound represented by the following Formula 2, a compound represented by the following Formula 3 or a mixture thereof:

[Formula 2]

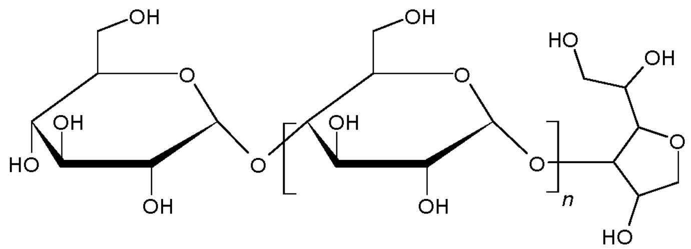

[Formula 3]

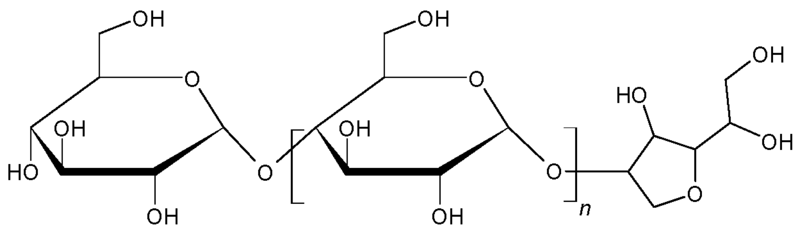

In Formulae 2 and 3, each of n is independently an integer of 0 to 4.

4. The polyol composition according to claim 1, wherein the monoanhydrosugar alcohol is monoanhydrosugar hexitol.

5. The polyol composition according to claim 1, wherein the dianhydrosugar alcohol is dianhydrosugar hexitol.

6. The polyol composition according to claim 1, wherein e) the polymer of one or more of a) to d) comprises at least one selected from the group consisting of condensation polymers prepared from the following condensation reaction:

- condensation reaction of monoanhydrosugar alcohol,
- condensation reaction of dianhydrosugar alcohol,
- condensation reaction of the polysaccharide alcohol represented by Formula 1,
- condensation reaction of anhydrosugar alcohol derived from polysaccharide alcohol represented by Formula 1,
- condensation reaction of monoanhydrosugar alcohol and dianhydrosugar alcohol,
- condensation reaction of monoanhydrosugar alcohol and polysaccharide alcohol represented by Formula 1,
- condensation reaction of monoanhydrosugar alcohol and anhydrosugar alcohol derived from polysaccharide alcohol represented by Formula 1,
- condensation reaction of dianhydrosugar alcohol and polysaccharide alcohol represented by Formula 1,
- condensation reaction of dianhydrosugar alcohol and anhydrosugar alcohol derived from polysaccharide alcohol represented by Formula 1,
- condensation reaction of polysaccharide alcohol represented by Formula 1 and anhydrosugar alcohol derived from polysaccharide alcohol represented by Formula 1,
- condensation reaction of monoanhydrosugar alcohol, dianhydrosugar alcohol and polysaccharide alcohol represented by Formula 1,
- condensation reaction of monoanhydrosugar alcohol, dianhydrosugar alcohol and anhydrosugar alcohol derived from polysaccharide alcohol represented by Formula 1,
- condensation reaction of monoanhydrosugar alcohol, polysaccharide alcohol represented by Formula 1 and anhydrosugar alcohol derived from polysaccharide alcohol represented by Formula 1,
- condensation reaction of dianhydrosugar alcohol, polysaccharide alcohol represented by Formula 1 and anhydrosugar alcohol derived from polysaccharide alcohol represented by Formula 1, or
- condensation reaction of monoanhydrosugar alcohol, dianhydrosugar alcohol, polysaccharide alcohol represented by Formula 1 and anhydrosugar alcohol derived from polysaccharide alcohol represented by Formula 1.

7. The polyol composition according to claim 1, wherein the anhydrosugar alcohol composition is prepared by hydrogenating a glucose-containing saccharide composition to prepare a hydrogenated sugar composition, heating the obtained hydrogenated sugar composition under an acid catalyst to a dehydration reaction by heating and conducting thin-film-distillation of the obtained dehydration reaction product.

8. The polyol composition according to claim 7, wherein the glucose-containing saccharide composition comprises 41 to 99.5 wt% of glucose based on the total weight of the glucose-containing saccharide composition.

9. The polyol composition according to claim 7, wherein the hydrogenation is carried out under a hydrogen pressure condition of 30 to 80 atm and a heating condition of 110 to 135°C, the dehydration reaction is carried out under a reduced pressure condition of 1 to 100 mmHg and a heating condition of 105 to 200°C and the thin-film-distillation is conducted under a reduced pressure condition of 2 mbar or less and a heating condition of 150 to 175°C.

10. A method for preparing a polyol composition comprising the step of performing an addition reaction of an anhydrosugar alcohol composition and an alkylene oxide,

wherein more than 50 parts by weight and less than 4,000 parts by weight of alkylene oxide is reacted per 100 parts by weight of the anhydrosugar alcohol composition in the addition reaction, and
the anhydrosugar alcohol composition comprises a) monoanhydrosugar alcohol; b) dianhydrosugar alcohol; c) polysaccharide alcohol represented by the following Formula 1; d) anhydrosugar alcohol derived from the polysaccharide alcohol represented by the following Formula 1; and e) a polymer of one or more of a) to d):

[Formula 1]

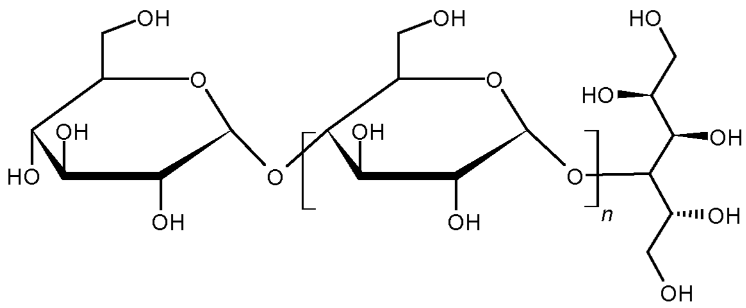

In Formula 1, n is an integer of 0 to 4.

**11.** A polyurethane prepolymer prepared by a reaction of the polyol composition according to any of claims 1 to 9 with a polyisocyanate.

**12.** A chain-extended polyurethane prepared by a reaction of the polyurethane prepolymer of claim 11 with a chain extender.

**13.** The chain-extended polyurethane according to claim 12, wherein the chain extender is selected from the group consisting of 1,4-butanediol, isosorbide, hydrazine monohydrate, ethylene diamine, dimethyl hydrazine, 1,6-hexamethylene bishydrazine, hexamethylene diamine, isophorone diamine, diaminophenylmethane or combinations thereof.

**14.** A method for preparing a chain-extended polyurethane comprising

(1) preparing a polyurethane prepolymer by reacting the polyol composition according to any of claims 1 to 9 with a polyisocyanate; and
(2) reacting the polyurethane prepolymer with a chain extender.

**15.** A hot-melt adhesive comprising the chain-extended polyurethane according to claim 12.

**INTERNATIONAL SEARCH REPORT**

International application No.
**PCT/KR2021/008638**

**A. CLASSIFICATION OF SUBJECT MATTER**

**C07H 15/08**(2006.01)i; **C07H 17/04**(2006.01)i; **C07H 1/00**(2006.01)i; **C08G 18/32**(2006.01)i; **C08G 65/26**(2006.01)i; **C08G 18/48**(2006.01)i; **C08G 18/10**(2006.01)i; **C08G 18/64**(2006.01)i; **C09J 175/04**(2006.01)i

According to International Patent Classification (IPC) or to both national classification and IPC

**B. FIELDS SEARCHED**

Minimum documentation searched (classification system followed by classification symbols)

C07H 15/08(2006.01); B01J 23/755(2006.01); B01J 27/04(2006.01); B01J 27/053(2006.01); C07D 493/04(2006.01); C08G 18/10(2006.01); C08G 18/40(2006.01); C08G 18/48(2006.01); C08G 65/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 알킬렌 옥사이드(alkylene oxide), 무수당 알코올(anhydrosugar alcohol), 폴리우레탄(polyurethane), 핫멜트 접착제(hot-melt adhesive)

**C. DOCUMENTS CONSIDERED TO BE RELEVANT**

| Category* | Citation of document, with indication, where appropriate, of the relevant passages | Relevant to claim No. |
|---|---|---|
| A | KR 10-2019-0137483 A (SAMYANG CORPORATION) 11 December 2019 (2019-12-11) See claims 1 and 2; and paragraph [0018]. | 1-15 |
| A | KR 10-2014-0080749 A (SAMYANG GENEX CORPORATION) 01 July 2014 (2014-07-01) See claims 1-13. | 1-15 |
| A | KR 10-2019-0000419 A (SAMYANG CORPORATION) 03 January 2019 (2019-01-03) See claims 1-13. | 1-15 |
| A | JP 09-194588 A (MITSUI TOATSU CHEM. INC.) 29 July 1997 (1997-07-29) See entire document. | 1-15 |
| A | JP 2013-142128 A (DAI ICHI KOGYO SEIYAKU CO., LTD.) 22 July 2013 (2013-07-22) See entire document. | 1-15 |

✓ Further documents are listed in the continuation of Box C.    ✓ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 October 2021** | **12 October 2021** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2021/008638**

**C. DOCUMENTS CONSIDERED TO BE RELEVANT**

| Category* | Citation of document, with indication, where appropriate, of the relevant passages | Relevant to claim No. |
|---|---|---|
| A | KR 10-2014-0105185 A (SAMYANG GENEX CORPORATION) 01 September 2014 (2014-09-01) See entire document. | 1-15 |
| A | KR 10-2017-0137235 A (SAMYANG CORPORATION) 13 December 2017 (2017-12-13) See entire document. | 1-15 |

**INTERNATIONAL SEARCH REPORT**
**Information on patent family members**

International application No.
**PCT/KR2021/008638**

| Patent document cited in search report | Publication date (day/month/year) | Patent family member(s) | Publication date (day/month/year) |
|---|---|---|---|
| KR 10-2019-0137483 A | 11 December 2019 | KR 10-2212004 B1 | 05 February 2021 |
| KR 10-2014-0080749 A | 01 July 2014 | CN 104854109 A | 19 August 2015 |
| | | CN 104854109 B | 26 April 2017 |
| | | EP 2933256 A1 | 21 October 2015 |
| | | EP 2933256 A4 | 20 July 2016 |
| | | EP 2933256 B1 | 22 May 2019 |
| | | JP 2016-501893 A | 21 January 2016 |
| | | JP 6097406 B2 | 15 March 2017 |
| | | KR 10-1475388 B1 | 23 December 2014 |
| | | US 2015-0329448 A1 | 19 November 2015 |
| | | US 9169180 B1 | 27 October 2015 |
| | | WO 2014-092490 A1 | 19 June 2014 |
| KR 10-2019-0000419 A | 03 January 2019 | CN 110799561 A | 14 February 2020 |
| | | EP 3643732 A1 | 29 April 2020 |
| | | JP 2020-524732 A | 20 August 2020 |
| | | JP 2021-098865 A | 01 July 2021 |
| | | KR 10-1943877 B1 | 31 January 2019 |
| | | KR 10-1988466 B1 | 13 June 2019 |
| | | KR 10-1989961 B1 | 17 June 2019 |
| | | KR 10-2019-0001621 A | 07 January 2019 |
| | | KR 10-2019-0001622 A | 07 January 2019 |
| | | US 2021-0147609 A1 | 20 May 2021 |
| | | WO 2018-236192 A1 | 27 December 2018 |
| JP 09-194588 A | 29 July 1997 | None | |
| JP 2013-142128 A | 22 July 2013 | JP 5809989 B2 | 11 November 2015 |
| KR 10-2014-0105185 A | 01 September 2014 | None | |
| KR 10-2017-0137235 A | 13 December 2017 | KR 10-1822838 B1 | 30 January 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020130119850
- KR 101370442
- KR 101709909
- KR 101079518
- KR 1020120066904